# EUROPEAN PATENT APPLICATION

(11) **EP 4 215 972 A1**
(43) Date of publication of application: **26.07.2023**
(21) Application number: 22216740.5
(22) Date of filing: 27.12.2022
(51) Int. Cl.: G02B 21/00, G01N 21/47, G01B 9/02091, C12M 1/34, G01N 15/00, G06V 20/69, G06T 7/00, G01N 33/483, G01N 15/14, G01N 21/25, G01N 21/64

(54) **IMAGE DISPLAY METHOD, IMAGE DISPLAY DEVICE, PROGRAM AND COMPUTER-READABLE RECORDING MEDIUM**

(30) Priority: 19.01.2022 JP 2022006549
(71) Applicant: SCREEN Holdings Co., Ltd., Kyoto 602-8585 (JP)
(72) Inventor: TOKISUE, Shogo, Kyoto-shi, 602 8585 (JP); HASEBE, Ryo, Kyoto-shi, 602 8585 (JP); OGI, Hiroshi, Kyoto-shi, 602 8585 (JP)
(74) Representative: Goddar, Heinz J.

(57) **Abstract**

An image display method includes the following operations (a) to (e). The (a) is of obtaining a plurality of two-dimensional images by two-dimensionally imaging a specimen, in which a plurality of objects to be observed are present three-dimensionally in the specimen, at a plurality of mutually different focus positions. The (b) is of obtaining image data representing a three-dimensional shape of the specimen. The (c) is of obtaining a three-dimensional image of the specimen based on the image data. The (d) is of obtaining the two-dimensional image selected from the plurality of two-dimensional images or a two-dimensional image generated to be focused on the plurality of objects based on the plurality of two-dimensional images as an integration two-dimensional image. The (e) is of integrating the integration two-dimensional image obtained in the (d) with the three-dimensional image obtained in the (c) and displaying an integrated image on a display unit.

## Description

### CROSS REFERENCE TO RELATED APPLICATION

The disclosure of Japanese Patent Application No. 2022-006549 filed on January 19, 2022 including specification, drawings and claims is incorporated herein by reference in its entirety.

### BACKGROUND OF THE INVENTION

### 1. Field of the Invention

This invention relates to a technique for displaying an image obtained by imaging a specimen in which a plurality of objects to be observed are present three-dimensionally.

### 2. Description of the Related Art

In pathological medicine and cell culture, two-dimensional observation and three-dimensional observation are performed to judge whether objects to be observed such as three-dimensionally cultured cells and tissue sections are good or bad and developmental statuses of these objects. Two-dimensional observation is mainly performed using an optical microscope. On the other hand, three-dimensional observation is performed using optical coherence tomography (OCT) or a three-dimensional observation equipment such as a confocal microscope or X-ray observation device. Here, if an invention described in JP 2020-524061A is applied, two-dimensional observation and three-dimensional observation can be integrated. By this integrated image, the convenience and understanding of an observer can be enhanced.

### SUMMARY OF THE INVENTION

The invention described in JP 2020-524061A aims to support a surgical operation when a surgeon performs a surgical procedure on an eye, and an optical microscope for obtaining an optical image with a surface of the eye focused and an OCT unit for obtaining an OCT image of a target position are optically coupled. The OCT image is superimposed on a single optical image obtained by the optical microscope. Therefore, in the case of observing a specimen in which a plurality of objects to be observed such as cells or tissue sections are present three-dimensionally, some objects to be observed may be clearly displayed, but the remaining objects to be observed may be unclear.

This invention was developed in view of the above problem and aims to provide an image display technique for enabling satisfactory observation of a specimen in which a plurality of objects to be observed are present three-dimensionally.

A first aspect of the invention is an image display method. The image display method comprises: (a) obtaining a plurality of two-dimensional images by two-dimensionally imaging a specimen, in which a plurality of objects to be observed are present three-dimensionally, at a plurality of mutually different focus positions; (b) obtaining image data representing a three-dimensional shape of the specimen; (c) obtaining a three-dimensional image of the specimen based on the image data; (d) obtaining the two-dimensional image selected from the plurality of two-dimensional images or a two-dimensional image generated to be focused on the plurality of objects to be observed based on the plurality of two-dimensional images as an integration two-dimensional image; and (e) integrating the integration two-dimensional image obtained in the operation (d) with the three-dimensional image obtained in the operation (c) and displaying an integrated image on a display unit.

A second aspect of the invention is an image display device. The image display device comprises: a two-dimensional imager configured to two-dimensionally image a specimen, in which a plurality of objects to be observed are present three-dimensionally, at a plurality of mutually different focus positions; a three-dimensional image acquirer configured to obtain a three-dimensional image of the specimen based on image data obtained by imaging the specimen and representing a three-dimensional shape of the specimen; a two-dimensional image acquirer configured to obtain a two-dimensional image selected from a plurality of two-dimensional images obtained by two-dimensional imaging by the two-dimensional imager or a two-dimensional image generated to be focused on the plurality of objects to be observed based on the plurality of two-dimensional images as an integration two-dimensional image; an integrated image generator configured to generate an integrated image by integrating the integration two-dimensional image with the three-dimensional image; and a display unit configured to display the integrated image.

A third aspect of the invention is a program for causing a computer to perform the operations (a) to (e) and a computer-readable recording medium, non-temporarily recording the program. The above method is suitable for execution by a computer device, and by realizing it as a program, it is possible to display the sample on the display unit using existing hardware resources.

In the invention thus configured, a plurality of two-dimensional images of the same specimen are obtained. These two-dimensional images are obtained by two-dimensional imaging at a plurality of mutual different focus positions. Further, image data representing a three-dimensional shape of the same specimen as the two-dimensionally imaged specimen is obtained, and a three-dimensional image of the specimen is obtained based on this image data. After the three-dimensional image and the plurality of two-dimensional images are obtained, an integration two-dimensional image selected or generated from the plurality of two-dimensional images is integrated with the three-dimensional image and an integrated image is displayed on the display unit. Therefore, not only merely the three-dimensional image, but also the integration two-dimensional image corresponding to the three-dimensional image are displayed in combination on the display unit for the specimen in which a plurality of objects to be observed are present three-dimensionally. As a result, an operator understands the specimen more highly through the integrated image displayed on the display unit.

As described above, according to the invention, the specimen in which the plurality of objects to be observed are present three-dimensionally can be satisfactorily observed.

All of a plurality of constituent elements of each aspect of the invention described above are not essential and some of the plurality of constituent elements can be appropriately changed, deleted, replaced by other new constituent elements or have limited contents partially deleted in order to solve some or all of the aforementioned problems or to achieve some or all of effects described in this specification. Further, some or all of technical features included in one aspect of the invention described above can be combined with some or all of technical features included in another aspect of the invention described above to obtain one independent form of the invention in order to solve some or all of the aforementioned problems or to achieve some or all of the effects described in this specification.

### BRIEF DESCRIPTION OF THE DRAWINGS

FIG. 1 shows a configuration example of an image processing apparatus equipped with a first embodiment of an image display device according to the invention.
FIG. 2 is a flow chart of a processing performed in the image processing apparatus shown in FIG. 1.
FIG. 3 is a diagram showing an example of a two-dimensional image group obtained in the image processing apparatus.
FIG. 4 is a flow chart of the image display processing corresponding to the first embodiment of the image display method according to the invention.
FIG. 5 is a flow chart showing an example of a horizontal alignment operation for aligning the integration two-dimensional image and the three-dimensional image in the horizontal direction.
FIG. 6 is a flow chart showing an example of a vertical alignment operation for aligning the integration two-dimensional image and the three-dimensional image in the vertical direction.
FIG. 7 is a flow chart of an image display processing corresponding to a second embodiment of the image display method according to the invention.
FIG. 8 is a flow chart of an image display processing corresponding to a third embodiment of the image display method according to the invention.
FIG. 9 is a flow chart of an image display processing corresponding to a fourth embodiment of the image display method according to the invention.

### DETAILED DESCRIPTION OF THE PREFERRED EMBODIMENTS

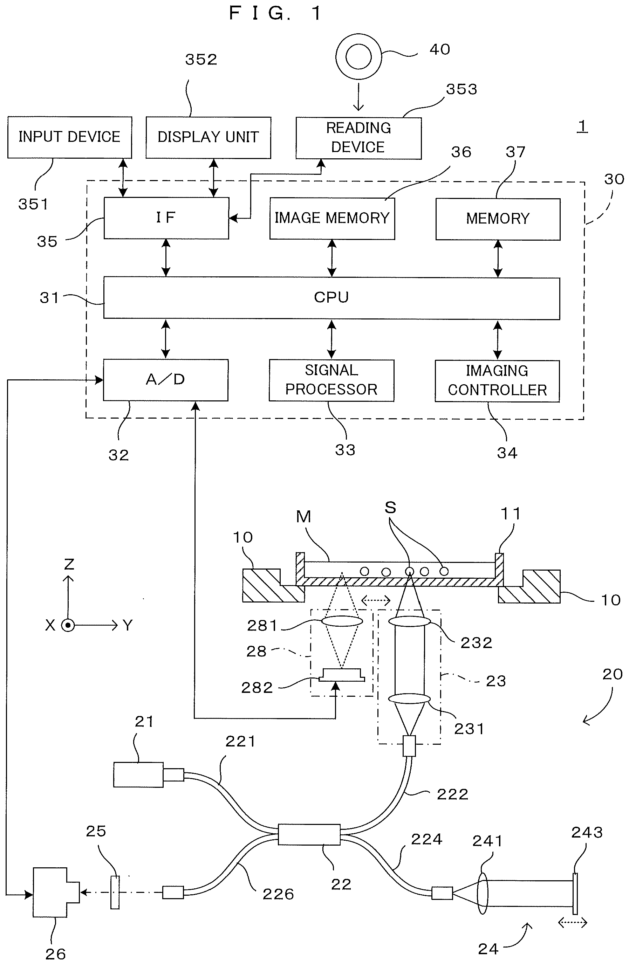

FIG. 1 shows a configuration example of an image processing apparatus equipped with a first embodiment of an image display device according to the invention. This image processing apparatus 1 performs tomography in parallel with the capture of two-dimensional images of a specimen carried in a liquid such as an embryo (fertilized egg) at mutually different focus positions. The image processing apparatus 1 also generates a stereoscopic image (three-dimensional image) of the specimen from a plurality of obtained pieces of tomographic image data, integrates the two-dimensional image selected from a plurality of the two-dimensional images with the stereoscopic image and displays an integrated image. Note that although an example of imaging a spheroid in the culture medium as the imaging object is illustrated here, the imaging object is not limited to this. For unified presentation of the directions in drawings, the XYZ orthogonal coordinate axes are established as shown in FIG. 1. The XY plane is a horizontal surface. The Z axis represents the vertical axis, in more detail, the (-Z) direction represents the vertically downward direction.

The image processing apparatus 1 includes a holder 10. The holder 10 holds a specimen container 11 containing specimens S serving as imaging objects in a horizontal posture. The specimen container 11 is, for example, a flat container in the form of a shallow plate called a dish formed with a recess capable of carrying the liquid on the upper surface of a plate-like member. A culture medium M such as a culture solution is poured into the specimen container 11, and fertilized eggs serving as the specimens are carried inside.

Although the plurality of specimens S are carried in the specimen container 11 having the single recess in this example, there is no limitation to this. For example, the specimen container 11 may be a well plate in which a plurality of recesses called wells are arranged in one plate-like member. In this case, one each of the plurality of specimens S can be carried in each of the plurality of wells. Further, for example, a plurality of dishes each carrying the specimen S may be held by the holder 10 while being arranged in a horizontal direction, and imaged.

The imager 20 is arranged below the container 11 held by the holder 10. An OCT device capable of capturing a tomographic image of an imaging object in a noncontact and non-destructive (non-invasive) manner is used as the imager 20. As described in detail later, the imager 20, which is an OCT device, includes a light source 21 for generating illumination light to the imaging object, an optical fiber coupler 22, an objective optical system 23, a reference optical system 24, a spectroscope 25 and a photo-detector 26.

The imager 20 further includes a microscopic imaging unit 28 for two-dimensional imaging with an optical microscope. More specifically, the microscopic imaging unit 28 includes an imaging optical system 281 and an imaging element 282. The imaging optical system 281 includes an objective lens, and the objective lens is focused on the sample S in the sample container 11. For example, a CCD imaging element, a CMOS sensor or the like can be, for example, used as the imaging element 282. The microscopic imaging unit 28 is preferably capable of bright field imaging or phase difference image. The objective optical system 23 and the microscopic imaging unit 28 are supported by a support member (not shown) movable in a horizontal direction and the positions thereof in the horizontal direction can be changed. Thus, in this embodiment, the microscopic imaging unit 28 corresponds to an example of the "two-dimensional imager" of the invention.

The image processing apparatus 1 further comprises a control unit 30 which controls an operation of the apparatus and a driving mechanism (not shown) which drives movable parts of the imager 20. The control unit 3 includes a CPU (Central Processing Unit) 31, an A/D convertor 32, a signal processor 33, an imaging controller 34, an interface (I/F) section 35, an image memory 36 and a memory 37.

The CPU 31 governs operations of the entire apparatus by executing a predetermined control program, thereby realizes various processing described later. The control program executed by the CPU 301 and data which are generated during processing are stored in the memory 37. The A/D convertor 32 converts signals which the photo-detector 26 and the imaging element 282 of the imager 20 output in accordance with the amount of received light into digital image data. The signal processor 33 performs image processing described later based upon a digital data outputted from the A/D convertor 32, thereby generates various images such as the tomographic image and 3D image of the imaging object. The image memory 36 saves the image data thus generated.

The imaging controller 34 controls the imager 20 to execute imaging process. Specifically, the imaging controller 34 set the objective optical system 23 for tomographic imaging and the microscopic imaging unit 28 selectively to an imaging position where the specimen S to be imaged is included in an imaging field of view. When the objective optical system 23 is positioned at the imaging position, the imaging controller 34 causes the imager 20 to execute an OCT imaging process described later for obtaining 3D image data indicating a solid structure, i.e. three-dimensional shape of the specimen S. On the other hand, when the microscopic imaging unit 28 is positioned at the imaging position, the imaging controller 34 causes the imager 20 causes the microscopic imaging unit 28 to obtain 2D image data corresponding to a planar image of the specimen S formed on a receiving surface of the imaging element 282. Note that the objective optical system 23 and the microscopic imaging unit 28 may be positioned relative to the specimen S to be imaged by moving only the holder 10 or moving both the holder 10 and the objective optical system 23 and the microscopic imaging unit 28 besides moving only the objective optical system 23 and the microscopic imaging unit 28 as in this embodiment.

The interface section 35 realizes communication between the image processing apparatus 1 and outside. More specifically, the interface section 35 has a function of communicating with external equipment, and a user interface function of accepting manipulation by a user and informing the user of various types of information. For achieving these objects, the interface section 35 comprises an input device 351 and a display unit 352. The input device 351 includes, for instance a key board, a mouse, a touch panel or the like which can accept manipulation and entry concerning selection of the functions of the apparatus, setting of operating conditions, etc. Further, the display unit 352 includes a liquid crystal display for example which shows various types of processing results such as the tomographic images and the 3D images generated by the imager 20. Further, to provide the above program from an apparatus outside, a reading device 353 for reading the above program from a computer-readable recording medium 40 (e.g. an optical disk, a magnetic disk, a magneto optical disk or the like) non-temporarily recording the above program may be connected to the interface section 35 as appropriate. In the case of using the recording medium 40, the CPU 31 reads the program from the recording medium 40 via the interface section 35 beforehand and expands the program in the memory 37. The CPU 31 performs an arithmetic processing in accordance with the program stored in the memory 37 (i.e. the control unit 30 executes the program), whereby each component of the apparatus configured as described next is controlled. Note that the program can be implemented in the control unit 30 by being received via an electrical communication line besides being read from the recording medium 40.

In the imager 20, from the light source 21 which includes a light emitting element such as a light emitting diode or a super luminescent diode (SLD) for instance, a low-coherence light beam containing wide-range wavelength components is emitted. For imaging the specimen such as cells or the like, an infrared light can be used favorably to make illumination light penetrate into the specimen.

The light source 21 is connected one optical fiber 221 of optical fibers constituting the optical fiber coupler 22. Low-coherence light emitted from the light source 21 is branched into lights to two optical fibers 222, 224 by the optical fiber coupler 22. The optical fiber 222 constitutes an object side optical path. More specifically, light emitted from an end part of the optical fiber 222 is incident on an objective optical system 23.

The objective optical system 23 includes a collimator lens 231 and an objective lens 232. Light emitted from an end part of the optical fiber 222 is incident on the objective lens 232 via the collimator lens 231. The objective lens 232 has a function of converging light (observation light) from the light source 21 to the specimen and a function of condensing reflected light from the specimen and causing the condensed reflected light toward the optical fiber coupler 22. Although a single objective lens 232 is shown in FIG. 1, a plurality of optical elements may be combined. Reflected light from the imaging object is incident as signal light on the optical fiber 222 via the objective lens 232 and the collimator lens 231. An optical axis of the objective lens 232 is orthogonal to the bottom surface of the container 11 and, in this example, an optical axis direction coincides with a vertical axis direction.

The CPU 31 sends a control command to the imaging controller 34. In response to the control command, the imaging controller 34 causes the imager 20 to move to a predetermined direction. More specifically, the imaging controller 34 makes the imager 20 move in a horizontal direction (XY direction) and a vertical direction (Z direction). By a movement of the imager 20 in the horizontal direction, the imaging field of view moves in the horizontal direction. Further, by a movement of the imager 20 in the vertical direction, a focus position of the objective optical system 23 along the optical axis direction changes relative to the specimen S as the imaging object.

Part of light incident on the optical fiber coupler 22 from the light source 21 is incident on the reference optical system 24 via an optical fiber 224. The reference optical system 24 includes a collimator lens 241 and a reference mirror 243. These constitute a reference system optical path together with the optical fiber 224. Specifically, light emitted from an end part of the optical fiber 224 is incident on the reference mirror 243 via the collimator lens 241. The light reflected by the reference mirror 243 is incident as reference light on the optical fiber 223.

The reference mirror 243 is supported by an advancing/retracting member (not shown). The advancing/retracting mechanism operates in response to a control command from the imaging controller 34, and includes an appropriate mechanism for advancing and retracting the reference mirror 243 in a Y direction, e.g. a linear motor or a ball screw mechanism. By moving the reference mirror 243 in Y direction, that is, a direction advancing to or retracting from the collimator lens 241, an optical path length of the reference light reflected by the reference mirror specimen 243 is adjusted.

The reflected light (signal light) reflected by a surface or an internal reflecting surface of the specimen and reference light reflected by the reference mirror 243 are mixed in the optical fiber coupler 22 and incident on the photo-detector 26 via the optical fiber 226. At this time, interference due to a phase difference between the reflected light and the reference light occurs, but an optical spectrum of interference light differs depending on a depth of the reflecting surface. That is, the optical spectrum of the interference light has information on a depth direction of the imaging object. Thus, a reflected light intensity distribution in the depth direction of the imaging object can be obtained by spectrally diffracting the interference light at each wavelength to detect a light quantity and Fourier transforming a detected interference signal. An OCT imaging technique based on such a principle is called Fourier domain OCT (FD-OCT).

The imager 20 of this embodiment is provided with a spectroscope 25 on an optical path of the interference light from the optical fiber 226 to the photo-detector 26. A spectroscope utilizing a prism, a spectroscope utilizing a diffraction grating and the like can be, for example, used as the spectroscope 25. The interference light is spectrally diffracted for each wavelength component and received by the photo-detector 26.

By Fourier-transforming the interference signal output from the photo-detector 26 according to the interference light detected by the photo-detector 26, the reflected light intensity distribution of the specimen in the depth direction, i.e. in the Z direction at the incident position of the illumination light is obtained. By scanning the illumination light incident on the container 11 in the X direction, the reflected light intensity distribution in a plane parallel to an XZ plane is obtained, with the result that a tomographic image of the specimen having this plane as a cross-section can be generated. A principle of generation of the tomographic image is not described because it is known.

Images are obtained by changing the incident position of the light along the Y direction over multiple steps and imaging a tomographic image for every change. By doing so, a number of tomographic images of the specimen are obtained along cross-sectional surfaces which are parallel to the XZ plane. As the scan pitch in the Y direction is reduced, it is possible to obtain image data with sufficient resolution to grasp the stereoscopic structure of the specimen. From these tomographic image data, 3D image data (e.g. voxel data, point cloud data or the like) corresponding to a body of the specimen can be obtained.

As just described, this image processing apparatus 1 has a function of obtaining an image of the specimen S carried together with the culture medium M in the container 11. More specifically, the image processing apparatus 1 is configured to be able to acquire two-dimensional image obtained by optical microscope imaging and three-dimensional image formed on the basis of tomographic image data obtained by OCT imaging.

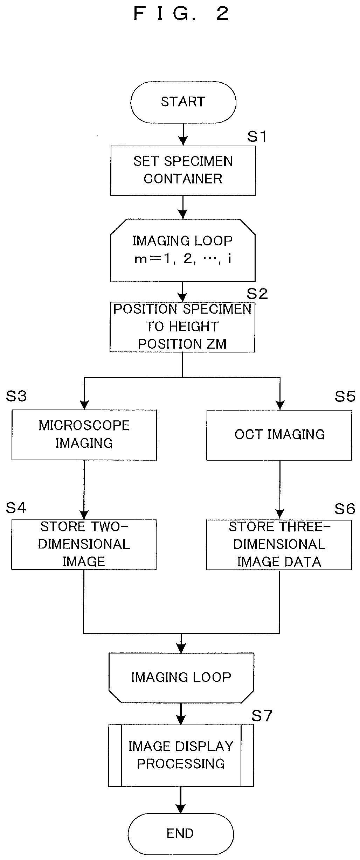

One mode of an image display processing executable using the image processing apparatus 1 configured as described above is described with reference to FIGS. 2 to 6. This image display processing corresponds to a first embodiment of image display method according to the invention. The image display processing in the first embodiment performs a step in which the CPU 31 obtains a three-dimensional image of the specimen S in accordance with the program stored in the memory 37, a step in which a two-dimensional image selected from a plurality of two-dimensional images is obtained as an integration two-dimensional image, and a step in which the integration two-dimensional image is integrated with the three-dimensional image to generate an integrated image. Then, the display unit 352 displays this integrated image. As just described, the CPU 31 functions as a "three-dimensional image acquirer", a "two-dimensional image acquirer" and an "integrated image generator" in this embodiment.

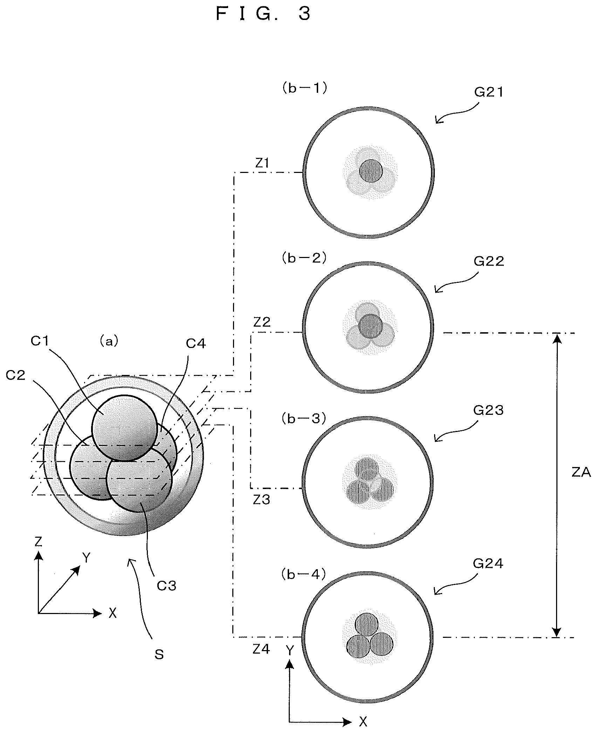

FIG. 2 is a flow chart of a processing performed in the image processing apparatus shown in FIG. 1. The first embodiment of the image display processing according to the invention is included in this processing. FIG. 3 is a diagram showing an example of a two-dimensional image group obtained in the image processing apparatus. This processing is realized based on image data obtained by the CPU 31 executing the program prepared in advance to cause each component of the apparatus to perform a predetermined operation and image the specimen S. If the specimen container 11 containing embryos to be evaluated is taken out from an incubator and set in the holder 10 (Step S1), imaging by the microscopic imaging unit 28 (hereinafter, referred to as "optical microscope imaging") and OCT imaging by the imager 20 are substantially simultaneously performed using the embryos as imaging objects. Note that "substantially simultaneously" mentioned here means to be within a period of cleavage and a case in which the optical microscope imaging and the OCT imaging are performed for the specimen S in a four-cell stage is illustrated in FIGS. 2 and 3.

Following Step S1, an imaging loop of performing the optical microscope imaging and the OCT imaging is entered. In this imaging loop, the optical microscope imaging and the OCT imaging are performed while the focus position is changed in multiple stages in a depth direction (Z direction). More specifically, the microscopic imaging unit 28 is positioned at an imaging position where the embryo to be evaluated can fall within an imaging field of view, and the specimen S is positioned to a height position Zm (Step S2). That is, in this imaging loop, the focus position is changed and set in multiple stages in the depth direction (Z direction).

Following positioning in the height direction, the microscopic imaging unit 28 obtains a two-dimensional image by imaging the embryo to be evaluated (Step S3) and sends image data of this two-dimensional image to the control unit 30. This image data is stored in the image memory 36. In this way, the two-dimensional image of the embryo included in the specimen S is stored (Step S4). A plurality of two-dimensional images G21 to G24 having mutually different focal depths, i.e. so-called Z stack images, are obtained, for example, as shown in FIG. 3 by microscope imaging in the imaging loop.

In parallel with this, the imager 20 performs the OCT imaging (Step S5). Three-dimensional image data obtained by this OCT imaging is sent to the control unit 30 and stored in the image memory 36 (Step S6).

If the imaging loop is repeated by the number of the stages set in advance, the CPU 31 exits from the imaging loop. Note that although two-dimensional imaging and three-dimensional imaging are performed in parallel in the imaging loop in this embodiment, imaging may be performed in two stages as long as a condition of being within the period of cleavage (i.e. while the specimen S is maintaining the same form) is satisfied. For example, the OCT imaging may be performed after two-dimensional imaging of the specimen S is performed. Of course, an imaging order may be switched.

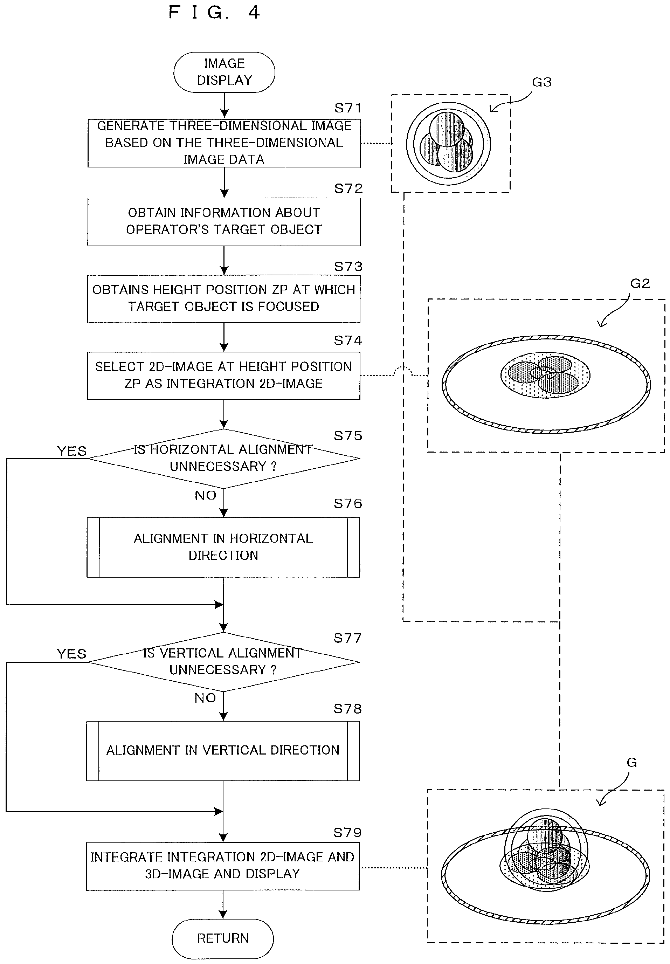

If the acquisition of the image data is completed, the CPU 31 performs an image display processing shown in FIG. 4 (Step S7).

FIG. 4 is a flow chart of the image display processing corresponding to the first embodiment of the image display method according to the invention. In this image display processing, a three-dimensional image G3 corresponding to the stereoscopic image of the specimen S is generated based on the three-dimensional image data stored in the image memory 36 (Step S71). Here, the three-dimensional image data constituting the three-dimensional image G3 may be point cloud data such as a polygon model or may be boxel data.

A two-dimensional image G2 to be integrated with the three-dimensional image G3 generated in this way (hereinafter, referred to as an "integration two-dimensional image") is selected from the plurality of two-dimensional images G21 to G24 shown in FIG. 3. This is because the embryo to be evaluated is divided into four cells C1 to C4 and the cells, boundaries between the cells and the like included in the two-dimensional images look different due to different focus positions as shown in FIG. 3. Accordingly, information on an object (specific cell or boundary between the cells) targeted by an operator is input via the input device 351. In this way, a targeted object is specified. Then, the CPU 31 obtains a height position Zp where the targeted object is in focus (Step S73). Note that since a method for finding out an in-focus position of the targeted object is known, this method is not described here. Further, if the height position Zp is directly input instead of inputting the information on the targeted object, Step S72 may be omitted.

In next Step S74, the two-dimensional image at the height position Zp is selected as the integration two-dimensional image. If the integration two-dimensional image G2 and the three-dimensional image G3 are prepared in this way, the both may be immediately integrated. However, the positions of the both images may be different due to characteristics and imaging environments of the imager 20 and the microscopic imaging unit 28. Accordingly, in this embodiment, alignment described next is performed in consideration of this point (Steps S75 to S78).

In a situation where alignment in the horizontal direction is unnecessary such as when the positions are strictly aligned by the imager 20 ("YES" in Step S75), alignment in the horizontal direction (Step S76) is unnecessary and Step S77 immediately follows. On the other hand, if the above situation is absent ("NO" in Step S75), alignment in the horizontal direction is performed (Step S76).

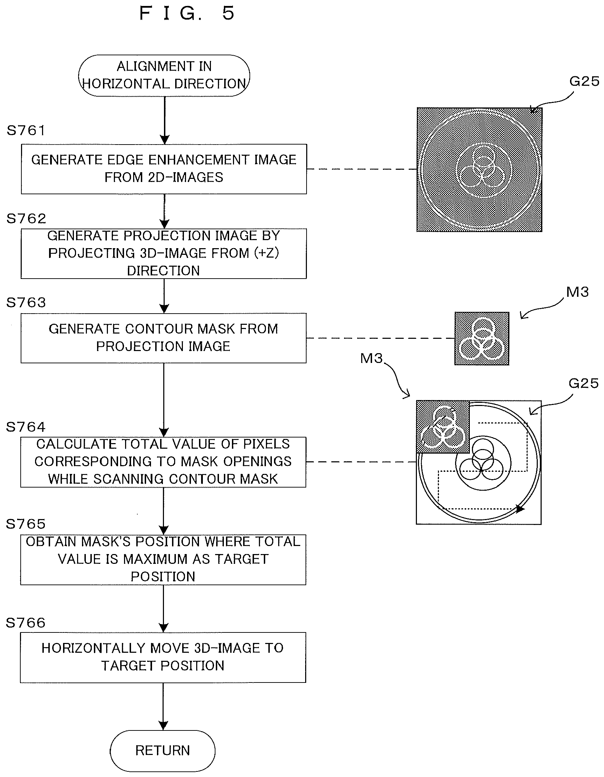

FIG. 5 is a flow chart showing an example of a horizontal alignment operation for aligning the integration two-dimensional image and the three-dimensional image in the horizontal direction. Here, position shift amounts in the horizontal direction are not directly obtained from the integration two-dimensional image G2 and the three-dimensional image G3, but another method is used. The CPU 31 generates an edge enhancement image G25 from all the two-dimensional images G21 to G24 (Step S761). In the generation of this edge enhancement image G25, various methods can be used. Here, two kinds of generation methods are illustrated and described. The first generation method includes the following steps (SA1) to (SA3).

(SA1) Such edge enhancement images that pixel values increase in parts where a pixel gradient is large are generated using an edge enhancement algorithm represented by a Laplacian filter for all the two-dimensional images G21 to G24. Note that, although not shown in figures, the edge enhancement images generated in this way are denoted by G'21 to G'24 for the convenience of description.

(SA2) Images in which the pixel values of the edge enhancement images G'21 to G'24 are maximum are selected for the respective pixel positions of the two-dimensional images.

(SA3) Pixel values of the two-dimensional images (images G21 to G24 respectively corresponding to the selected G'21 to G'24) corresponding to the images selected in the step (SA2) are generated by being set as the pixel values of the pixel positions.

Further, the second generation method has the following steps (SB1) to (SB3).

(SB1) Edge enhancement images G'21 to G'24 are generated in a manner similar to the step (SA1).

(SB2) A threshold value is arbitrarily set for pixel values of edges.

(SB3) The pixel value is identified as follows for each pixel position of the two-dimensional image. More specifically, if the pixel values of even one of the respective edge enhancement images G'21 to G'24 generated in the step (SB1) are equal to or more than the threshold value, the pixel values of the two-dimensional image corresponding to the edge enhancement image equal to or more than the threshold value are averaged. On the other hand, if the pixel values of the edge enhancement images G'21 to G'24 generated in the step (SB1) are all less than the threshold value identified in the step (SB2), the pixel values of all the two-dimensional images are averaged.

Further, the CPU 31 generates a projection image (not shown) by projecting the three-dimensional image G3 on a horizontal plane (XY plane) from the (+Z) direction (Step S762). Various generation means can be used for the generation of this projection image. For example, a generation method having the following steps (SC1) and (SC2) may be performed.

(SC1) A three-dimensional label (boxel data in which pixel values of a background are zero and pixel values of the label are positive values) is generated for each cell from the three-dimensional image.

(SC2) A contour mask having a boundary part of the label as a counter is obtained by the maximum value projection of the label image on the XY plane for each cell.

Then, the CPU 31 generates a contour mask M3 of the cells C1 to C4 by synthesizing the projection images, i.e. the contour masks of the respective cells obtained in the step (SC2) (Step S763).

In this contour mask M3, contour parts of the cells C1 to C4 are open. Thus, if the contour mask M3 is moved in the horizontal direction while being overlapped on the edge enhancement image G25, a degree of coincidence of the projection image with the edge enhancement image G25 changes according to that movement. The "degree of coincidence" mentioned here means a degree of overlap of the pixels representing the contours of the cells C1 to C4 included in the edge enhancement image G25 (hereinafter, referred to as "cell contour pixels") and the openings of the contour mask M3 (hereinafter, "mask openings"). A total value of the cell contour pixels located right below the mask openings changes according to a horizontal position of the contour mask M3. Particularly, the total value is maximized when the mask openings substantially coincide with the contours of the cells C1 to C4. Accordingly, in this embodiment, the CPU 31 calculates the total value of the cell contour pixels corresponding to the openings of the contour mask M3 while scanning the contour mask M3 in the horizontal direction with respect to the edge enhancement image G25 (Step S764). A position where the total value is maximum is obtained as a target position (Step S765). Note that the edge enhancement image G25 should be drawn similarly to a figure corresponding to Step 761 in a figure corresponding to this Step S764, but the edge enhancement image G25 is intentionally black-and-white reversed to clarify a relationship with the contour mask M3.

Further, although the contour mask M3 is used to obtain the target position in this embodiment, a contour mask of the specimen S composed of the cells C1 to C4 may be used. Further, besides a method using a mask, a position shift may be calculated by a known registration method using a two-dimensional image for registration generated from a plurality of two-dimensional images and a three-dimensional image for registration generated from a three-dimensional image and a target position may be obtained based on the calculated position shift.

In next Step S766, the CPU 31 moves the three-dimensional image G3 in the horizontal direction so that the three-dimensional image G3 is located at the target position. In this way, the integration two-dimensional image G2 and the three-dimensional image G3 overlap when viewed from the (+Z) direction and alignment in the horizontal direction is completed. Note that although alignment is performed by moving the three-dimensional image G3 in this embodiment, alignment in the horizontal direction may be performed by moving only the integration two-dimensional image G2 or moving both images.

Referring back to FIG. 4, the description is continued. Although alignment in the horizontal direction is described above, alignment in the vertical direction is also similar. In a situation where alignment in the vertical direction is clearly unnecessary such as when the positions are strictly aligned by the imager 20 ("YES" in Step S77), alignment in the vertical direction (Step S78) is unnecessary and Step S79 immediately follows. On the other hand, if the above situation is absent ("NO" in Step S77), alignment in the vertical direction (Z direction) is performed (Step S78).

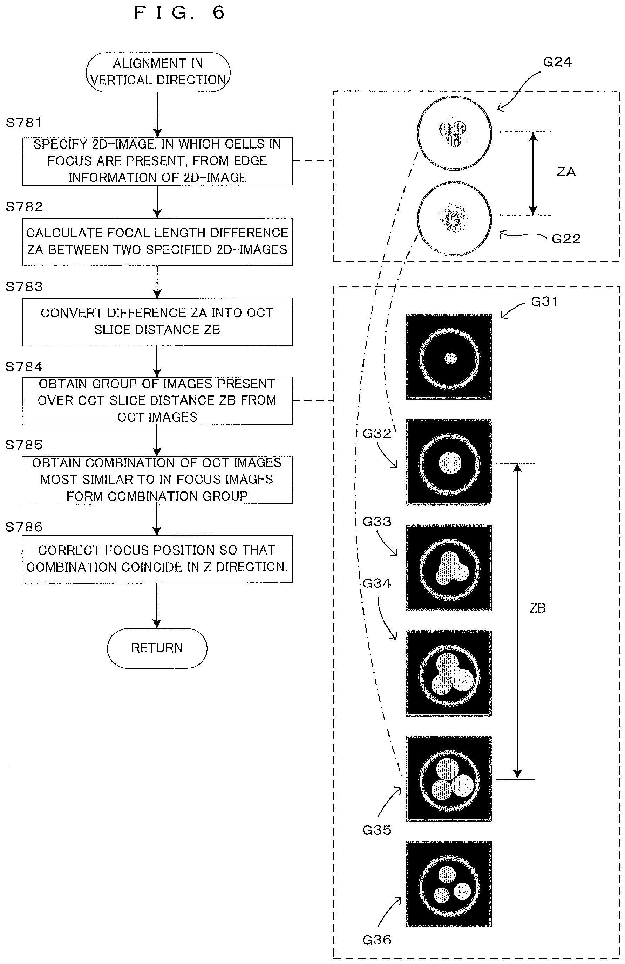

FIG. 6 is a flow chart showing an example of a vertical alignment operation for aligning the integration two-dimensional image and the three-dimensional image in the vertical direction. The CPU 31 obtains pieces of edge information of the two-dimensional images G21 to G24 and, then, specifies the two-dimensional image, in which the cells in focus are present, from those pieces of information (Step S781). For example, if the two-dimensional images G21 to G24 shown in FIG. 3 are obtained in Step S2, the two-dimensional image G22, in which an image of the cell C1 is clearly reflected, and the two-dimensional image G24, in which images of the cells C2 to C4 are clearly reflected, are specified in Step S781 as shown on a right upper side of FIG. 6.

The CPU 31 calculates a focal length difference ZA between these two two-dimensional images G22 and G24 (Step S782). The CPU 31 further converts the difference ZA into an OCT slice distance ZB (Step S783). This OCT slice distance ZB is a Z-direction slice difference (n-fold of a Z-direction scanning pitch) of OCT calculated from the focal length difference ZA.

The CPU 31 obtains a group of images present over the OCT slice distance ZB from OCT images. For example, OCT images G31 to G36 obtained by the OCT imaging are shown on a right lower side of FIG. 6. Note that although only six OCT images are shown in FIG. 6, the number of the actually captured OCT images is not limited to "6" and is arbitrary. Out of the group of these images, a combination most similar to the two-dimensional images G22, G24 is obtained (Step S785). For example, the OCT images having closest cell region areas and separated from each other by the OCT slice distance ZB may be selected as the most similar combination. Here, the description is continued, assuming that a combination (G32, G35) was selected in Step S785.

In next Step S786, the CPU 31 corrects the focus position so that the Z-direction positions of the combination of the selected OCT images (G32, G35) and those of the combination of the two-dimensional images (G22, G24) coincide.

In this way, the position shift in the Z direction between the integration two-dimensional image G2 and the three-dimensional image G3 is eliminated when viewed from the horizontal direction and alignment in the vertical direction is completed. Note that the alignment method is not limited to this and it goes without saying that another method may be used.

After alignment in the horizontal direction (Step S76) and alignment in the vertical direction (Step S78) are completed, the CPU 31 displays the integrated image G generated by integrating the integration two-dimensional image G2 and the three-dimensional image G3 on the display unit 352 by surface rendering (Step S79), for example, as shown on a right lower side of FIG. 4.

As described above, in this embodiment, the plurality of two-dimensional images G21 to G24 are obtained in consideration of the three-dimensional presence of the plurality of cells (objects to be observed) in the specimen S in generating the integrated image G by integrating the two-dimensional image and the three-dimensional image of the specimen S. For example, in the case of using a conventional technique, a two-dimensional image needs to be obtained again after a focus position is changed according to a cell of interest. In contrast, in this embodiment, a most suitable two-dimensional image, i.e. the integration two-dimensional image G2, is selected according to the cell of interest, and the integration two-dimensional image G2 and the three-dimensional image G3 are displayed in an integrated state on the display unit 352. Therefore, the specimen S can be satisfactorily observed in a short time. As a result, the operator viewing the integrated image G displayed on the display unit 352 can highly understand the specimen S.

Further, in this embodiment, before the integration two-dimensional image G2 and the three-dimensional image G3 are integrated, the both images G2, G3 are aligned in the horizontal direction and vertical direction. Thus, the integrated image G displayed on the display unit 352 further improves the understanding of the operator. Note that although the integration two-dimensional image G2 and the three-dimensional image G3 are three-dimensionally aligned in this embodiment, alignment may be performed in only one of the horizontal direction and vertical direction in terms of observation objects and observation purpose. Further, the alignment methods are not limited to those shown in FIGS. 5 and 6. Further, alignment is possibly not necessary in some cases.

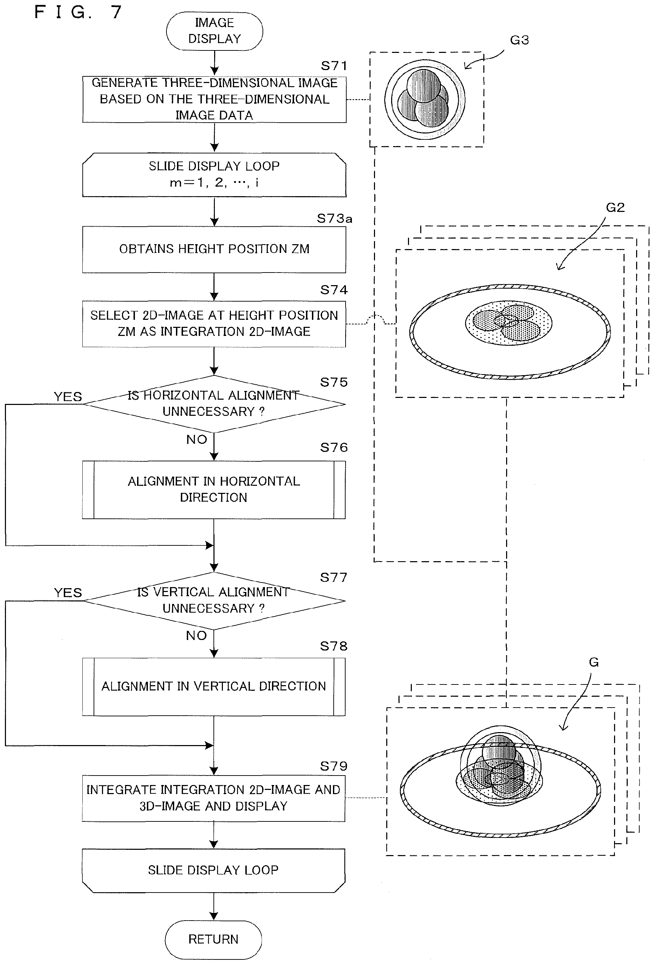

FIG. 7 is a flow chart of an image display processing corresponding to a second embodiment of the image display method according to the invention. The second embodiment largely differs from the first embodiment (FIG. 4) in that two-dimensional images G21 to G24 obtained at height positions Z1 to Z4 are respectively set as integration two-dimensional images G2, integrated with a three-dimensional image G3 and displayed without waiting for the input of information on a cell of interest. That is, in the second embodiment, after the three-dimensional image corresponding to a stereoscopic image of a specimen S is generated based on two-dimensional image data stored in the image memory 36 (Step S71), a slide display loop is entered in which the slide display of the integrated images G is carried out for each height position in an order of the height positions Z1 to Z4. In this slide display loop, the CPU 31 obtains a height position Zm (Step S73a). Then, in the same steps as the steps (Steps S74 to S79) of the first embodiment, the CPU 31 selects a two-dimensional image G2m obtained at the height position Zm as the integration two-dimensional image G2 and displays an integrated image G generated by integrating the integration two-dimensional image G2 and the three-dimensional image G3 on the display unit 352.

If the slide display loop (corresponding to an example of a "slide display step" of the invention) is repeated by the number of stages (four stages in this embodiment) set by such image display steps (S73a, S74 to S79) in advance, the CPU 31 exits from the slide display loop. Note that although the slide display loop is performed only once in the second embodiment, four kinds of the integrated images G may be stored in the image memory 36 and the slide display of these integrated images G on the display unit 352 may be carried out a plurality of times or repeatedly. Further, after or instead of the slide display, the four kinds of the integrated images G may be collectively displayed in a divided manner on the display unit 352. The collective divided display processing corresponds to an example of a "collective display step" of the invention. Further, the slide display or collective display may be stopped by the operator selecting the integrated image G from the four kinds of the integrated images G displayed on the display unit 352, and only the selected integrated image G may be displayed on the display unit 352.

As described above, according to the second embodiment, the operator can observe various integrated images G in turn or collectively. Further, a specific integrated image G can be displayed on the display unit 352 according to the operator's request. In this way, user friendly image display can be carried out.

Note that although alignment is performed in the horizontal direction and vertical direction for each of the height positions Z1, Z2 and so one in the second embodiment, Step S75 to S78 may be omitted by performing the alignment, for example, only for the first height position Z1 and using information on alignment at the height position Z 1 for the subsequent height positions Z2 and so on.

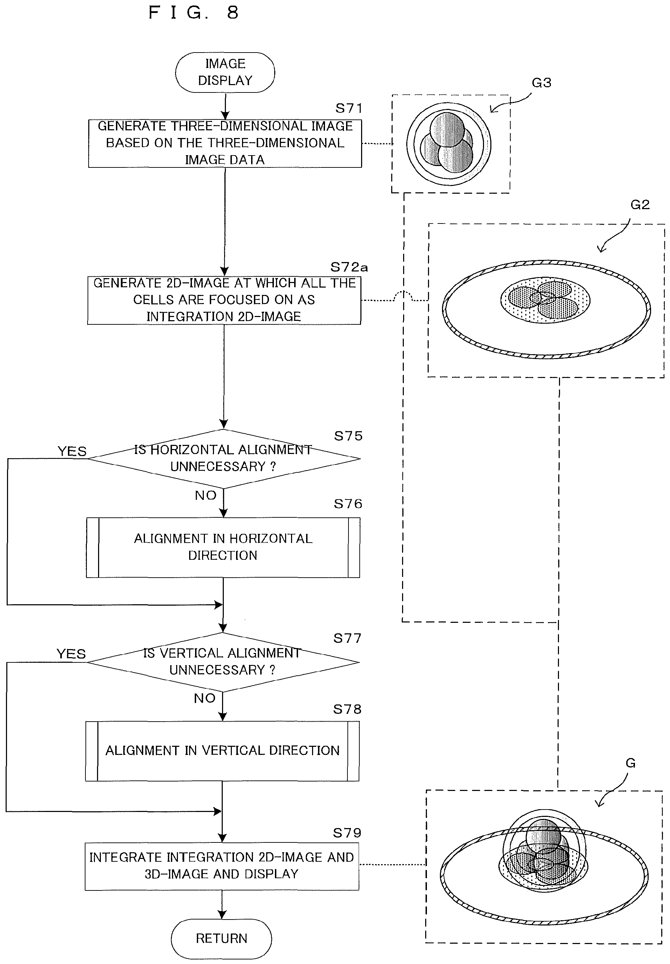

FIG. 8 is a flow chart of an image display processing corresponding to a third embodiment of the image display method according to the invention. The third embodiment largely differs from the first embodiment (FIG. 4) in the generation method of the integration two-dimensional image G2. That is, in the first embodiment, the integration two-dimensional image G2 is selected from the two-dimensional images G21 to G24 by performing the two-dimensional image selection steps (Steps S72 to S74). In contrast, in the third embodiment, a two-dimensional image generated to be focused on all the cells C1 to C4 based on all the two-dimensional images G21 to G24 is used as the integration two-dimensional image G2 (Step S72a) as shown in FIG. 8. The generation method having the steps (SA1) to (SA3) or the generation method having the steps (SB 1) to (SB3) for all the two-dimensional images G21 to G24 can be utilized as the generation method of this two-dimensional image.

The integration two-dimensional image G2 generated in this way and a three-dimensional image G3 are integrated by way of alignment in the horizontal direction and vertical direction (Steps S75 to S78) as in the first embodiment to generate an integrated image G. Then, this integrated image G is displayed on the display unit 352 (Step S79). However, since the integration two-dimensional image G2 is generated to be focused on all the cells C1 to C4 in the third embodiment, a degree of importance of alignment in the vertical direction is low. Therefore, alignment in the vertical direction may be actively omitted.

As described above, in the third embodiment, the integration two-dimensional image G2 focused on all the cells C1 to C4 and the three-dimensional image G3 are displayed in an integrated state on the display unit 352. Therefore, the specimen S can be satisfactorily observed in a short time. As a result, the operator viewing the integrated image G displayed on the display unit 352 can highly understand the specimen S.

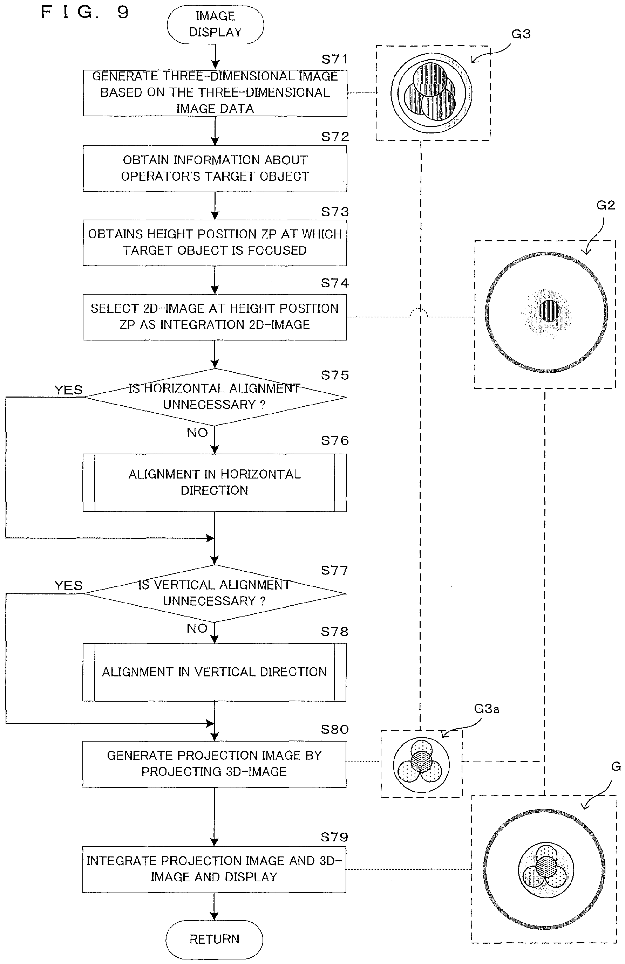

FIG. 9 is a flow chart of an image display processing corresponding to a fourth embodiment of the image display method according to the invention. The fourth embodiment largely differs from the first embodiment (FIG. 4) in that, instead of the two-dimensional image G3, a projection image G3a projected on a horizontal plane from the (+Z) direction is integrated with the integration two-dimensional image G2 and displayed. More specifically, as shown in FIG. 9, the projection image G3a is generated by projecting a three-dimensional image of the specimen S generated in Step S71 on a horizontal plane (XY plane) from a vertically upper side, i.e. from the (+Z) direction (Step S80). Various generation methods can be used for this projection image G3a. For example, the following steps (SD1) to (SD3) may be performed as an example of this generation method.

(SD1) A three-dimensional label (boxel data in which pixel values of a background are zero and pixel values of the label are positive values) is generated for each cell from the three-dimensional image.

(SD2) A contour mask is generated using a cell boundary part of each cell label (two-dimensional label image) in a cross-section of interest in the integration two-dimensional image G2 as a contour. However, if the integration two-dimensional image G2 is an image focused on all the cells, the contour mask is generated in a manner similar to the step SC2.

(SD3) The contour masks of the respective cells obtained in the step (SD2) are synthesized to generate the projection image G3a.

Then, this projection image 3Ga and the integration two-dimensional image G2 are integrated and displayed on the display unit 352. Note that the other configuration and operation are the same as in the first embodiment.

As described above, also in the fourth embodiment, the specimen S can be satisfactorily observed in a short time. As a result, the operator viewing the integrated image G displayed on the display unit 352 can confirm the certainty of the three-dimensional image G3.

Further, although the three-dimensional image is not displayed in the fourth embodiment, the following functions and effects are obtained by being used together with the first to third embodiments. That is, regions difficult to confirm only by the integration two-dimensional image G2, e.g. boundaries of the cells, may be present. In such a case, the boundaries of the cells and the like can be easily confirmed by displaying the integrated image of the fourth embodiment on the display unit 352. As just described, in the fourth embodiment, the projection image G3a has a function of supporting the integration two-dimensional image G2.

Further, although the projection image G3a is generated by projecting the three-dimensional image of the specimen S in the fourth embodiment, the projection image 3a may be generated by projecting only outer sides of the respective cell contours when the three-dimensional image is viewed from the (+Z) direction on the horizontal plane (XY plane).

Note that the invention is not limited to the above embodiment and various changes other than the aforementioned ones can be made without departing from the gist of the invention. For example, in the above embodiments, the integrated image G is displayed on the display unit 352 by surface rendering. Here, the operator may be able to interactively operate a viewpoint. Further, if it is difficult to superimpose and confirm the integration two-dimensional image 2G and the three-dimensional image G3, transparency may be given to either one of the images or only a frame may be displayed by deleting surfaces of a polygon model. That is, one of the integration two-dimensional image G2 and the three-dimensional image G3 may be displayed by surface rendering and the other may be displayed by volume rendering. In this way, the visibility of the integrated image G displayed on the display unit 352 can be enhanced.

Further, in the above embodiments, the image display device and the image display method according to the invention are incorporated into the image processing apparatus 1. However, the image display device and the image display method according to the invention may not have an imaging function themselves and can be carried out by a computer device having obtained imaging data obtained by imaging in another device having an imaging function.

Further, in the above embodiments, the embryo (fertilized egg) having the cells C1 to C4 divided by cleavage as objects to be observed is used as the specimen S and the integrated image G of this is displayed on the display unit 352. An application object of the invention is not limited to the observation of an embryo and can be applied to observations in general of specimens in which a plurality of objects to be observed are present three-dimensionally.

Further, although the optical microscope for bright field imaging or phase contrast imaging is used as the optical microscope for two-dimensionally imaging the specimen S in the above embodiments, a stereo microscope may be used besides these. Further, although the OCT device is used as the imager for three-dimensionally imaging the specimen S, a three-dimensional observation device, e.g. a fluorescent microscope, may be used besides this.

Although the invention has been described with reference to specific embodiments, this description is not meant to be construed in a limiting sense. Various modifications of the disclosed embodiment, as well as other embodiments of the present invention, will become apparent to persons skilled in the art upon reference to the description of the invention. It is therefore contemplated that the appended claims will cover any such modifications or embodiments as fall within the true scope of the invention.

This invention can be applied to techniques in general for displaying an image obtained by imaging a specimen in which a plurality of objects to be observed are present three-dimensionally.

## Claims

1. An image display method, comprising:
(a) obtaining a plurality of two-dimensional images by two-dimensionally imaging a specimen, in which a plurality of objects to be observed are present three-dimensionally, at a plurality of mutually different focus positions;
(b) obtaining image data representing a three-dimensional shape of the specimen;
(c) obtaining a three-dimensional image of the specimen based on the image data;
(d) obtaining the two-dimensional image selected from the plurality of two-dimensional images or a two-dimensional image generated to be focused on the plurality of objects to be observed based on the plurality of two-dimensional images as an integration two-dimensional image; and
(e) integrating the integration two-dimensional image obtained in the operation (d) with the three-dimensional image obtained in the operation (c) and displaying an integrated image on a display unit.

2. The image display method according to claim 1, wherein:
the operation (d) includes selecting one integration two-dimensional image from the plurality of two-dimensional images.

3. The image display method according to claim 2, wherein the operation (d) includes specifying an observation object from the plurality of objects to be observed and selecting the two-dimensional image focused on the object to be observed specified as the observation object as the integration two-dimensional image.

4. The image display method according to claim 1, wherein:
the operation (d) includes selecting the integration two-dimensional images one by one in turn from the plurality of two-dimensional images, and
the operation (e) includes a slide display step of integrating the selected integration two-dimensional image with the three-dimensional image and displaying the integrated image on the display unit every time the integration two-dimensional image is selected in the operation (d).

5. The image display method according to claim 1, wherein:
the operation (e) includes a step of generating a plurality of integrated images by integrating each two-dimensional image with the three-dimensional image for at least two or more of the plurality of two-dimensional images and a collective display step of displaying the plurality of integrated images on the display unit.

6. The image display method according to claim 1, wherein:
the operation (d) includes a step of generating the two-dimensional image focused on the plurality of objects to be observed as the integration two-dimensional image based on the plurality of two-dimensional images.

7. The image display method according to any one of claims 2 to 6, wherein:
the operation (e) includes a step of moving at least one of the integration two-dimensional image and the three-dimensional image in a vertical direction for alignment before the integration two-dimensional image is integrated with the three-dimensional image.

8. The image display method according to any one of claims 2 to 7, wherein:
the operation (e) includes a step of moving at least one of the integration two-dimensional image and the three-dimensional image in a plane parallel to the integration two-dimensional image for alignment before the integration two-dimensional image is integrated with the three-dimensional image.

9. The image display method according to any one of claims 1 to 8, comprising:
(f) integrating a projection image obtained by synthesizing contour masks of the respective objects to be observed generated from the three-dimensional image and the integration two-dimensional image and displaying an integrated image on the display unit.

10. The image display method according to any one of claims 1 to 9, wherein:
the plurality of three-dimensional images are constituted by point cloud data.

11. The image display method according to any one of claims 1 to 9, wherein:
the plurality of three-dimensional images are constituted only by a frame.

12. The image display method according to any one of claims 1 to 9, wherein:
the plurality of three-dimensional images are constituted by boxel data.

13. An image display device, comprising:
a two-dimensional imager configured to two-dimensionally image a specimen, in which a plurality of objects to be observed are present three-dimensionally, at a plurality of mutually different focus positions;
a three-dimensional image acquirer configured to obtain a three-dimensional image of the specimen based on image data obtained by imaging the specimen and representing a three-dimensional shape of the specimen;
a two-dimensional image acquirer configured to obtain a two-dimensional image selected from a plurality of two-dimensional images obtained by two-dimensional imaging by the two-dimensional imager or a two-dimensional image generated to be focused on the plurality of objects to be observed based on the plurality of two-dimensional images as an integration two-dimensional image;
an integrated image generator configured to generate an integrated image by integrating the integration two-dimensional image with the three-dimensional image; and
a display unit configured to display the integrated image.

14. A program for causing a computer to perform:
(a) obtaining a plurality of two-dimensional images by two-dimensionally imaging a specimen, in which a plurality of objects to be observed are present three-dimensionally, at a plurality of mutually different focus positions;
(b) obtaining image data representing a three-dimensional shape of the specimen;
(c) obtaining a three-dimensional image of the specimen based on the image data;
(d) obtaining the two-dimensional image selected from the plurality of two-dimensional images or a two-dimensional image generated to be focused on the plurality of objects to be observed based on the plurality of two-dimensional images as an integration two-dimensional image; and
(e) integrating the integration two-dimensional image obtained in the operation (d) with the three-dimensional image obtained in the operation (c) and displaying an integrated image on a display unit.

15. A computer-readable recording medium, non-temporarily recording the program according to claim 14.
